(19) Europäisches Patentamt
European Patent Office
Office européen des brevets

(11) **EP 4 238 591 A1**

(12) **EUROPEAN PATENT APPLICATION**
published in accordance with Art. 153(4) EPC

(43) Date of publication:
**06.09.2023 Bulletin 2023/36**

(21) Application number: **21886315.7**

(22) Date of filing: **28.10.2021**

(51) International Patent Classification (IPC):
**A61L 27/14** (2006.01)  **A61L 27/18** (2006.01)
**A61L 27/40** (2006.01)  **A61L 27/52** (2006.01)
**A61L 27/56** (2006.01)

(52) Cooperative Patent Classification (CPC):
**A61L 27/14; A61L 27/18; A61L 27/40; A61L 27/52;
A61L 27/56**

(86) International application number:
**PCT/JP2021/039818**

(87) International publication number:
**WO 2022/092198 (05.05.2022 Gazette 2022/18)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **28.10.2020 JP 2020180919**

(71) Applicant: **Kuraray Co., Ltd.
Kurashiki-shi, Okayama 710-0801 (JP)**

(72) Inventors:
• **KOBAYASHI, Goro
Tsukuba-shi, Ibaraki 305-0841 (JP)**
• **KAWAGOE, Masako
Osaka-shi, Osaka 530-8611 (JP)**
• **AYANO, Satoru
Tsukuba-shi, Ibaraki 305-0841 (JP)**

(74) Representative: **Müller-Boré & Partner
Patentanwälte PartG mbB
Friedenheimer Brücke 21
80639 München (DE)**

(54) **IMMUNOISOLATION DEVICE**

(57) An object of this invention relates to provide a macroencapsulation immunoisolation device that can be easily removed, does not break, and is suitable for improvement in the diffusion efficiency of a physiologically active substance required for transplantation while maintaining an immunoisolation effect. The present invention provide an immunoisolation device comprising an embedding chamber for a material to be transplanted, the embedding chamber being covered with an immunoisolation membrane.

Fig.3

Non-woven fabric

Porous membrane

Hydrogel

EP 4 238 591 A1

**Description**

Technical Field

Cross-Reference to Related Application

This application claims priority to Japanese Patent Application No. 2020-180919 filed on October 28, 2020 (the disclosure of which is incorporated herein by reference). The present invention relates to an immunoisolation device.

Background Art

Immunoisolation devices have been developed as a means for performing cell transplantation therapy without the need to administer an immunosuppressant. In particular, in transplantation of somatic cells derived from iPS cells, for which the risk of canceration is a concern, a decrease in the function of transplanted cells, or the like, macroencapsulation immunoisolation devices are considered an effective method in that the transplantation site can be identified and that the devices can be replaced.
Important functions required as macroencapsulation immunoisolation devices are the following: cells or cell clusters can be uniformly fixed in a dispersed state without aggregation; oxygen and/or nutritional components are allowed to easily permeate transplanted cells; desired physiologically active substances (cytokines, hormones, growth factors, etc.) that are released by cells and are required for a therapeutic effect can easily be released according to the cell response, and permeation of immunoresponsive cells and immune response factors can be prevented; and the transplanted devices are excellent in biocompatibility, and are less likely to adhere to surrounding tissue and less likely to induce inflammatory responses, such as granulation.
Many immunoisolation devices using porous membranes have been studied, but have problems such as a decrease in permeability due to protein adsorption to porous membrane materials, a decrease in permeability due to fibrosis, and a decrease in permeability due to adhesion to surrounding tissue.
Internal necrosis of cell clusters etc. is considered to be induced when the diameter exceeds 500 um. In order to easily maintain engraftment of embedded cells to a recipient and release physiologically active substances, it is necessary to appropriately control the overall thickness of such a device, and it is desirable that the immunoisolation membrane constituting the device is as thin as possible. On the other hand, for transplantation into the body, it is necessary to improve the strength and durability of the membrane to prevent the device from breaking or being twisted. However, it is not easy to achieve both a reduction in the membrane thickness and improvement in durability.

Summary of Invention

Technical Problem

An object of the present invention is to provide a macroencapsulation immunoisolation device that can be easily removed, does not break, and is suitable for improvement in the diffusion efficiency of a physiologically active substance required for transplantation while maintaining an immunoisolation effect, in long-term transplantation.
Another object of the present invention is to provide an invention that achieves excellent biocompatibility without interfering with engraftment of a material to be transplanted, such as cells and cell clusters, and simultaneously achieves a reduction in the diffusion distance in a device and improvement in durability while maintaining immunoisolation properties.

Solution to Problem

The present invention provides the following immunoisolation device.

[1] An immunoisolation device comprising an embedding chamber for a material to be transplanted, the embedding chamber being covered with an immunoisolation membrane.
[2] The immunoisolation device according to [1], wherein the immunoisolation membrane comprises at least two members selected from the group consisting of fiber structures, porous membranes, and hydrogels.
[3] The immunoisolation device according to [1], wherein the immunoisolation membrane is a multilayer membrane comprising at least two layers selected from the group consisting of fiber structure layers, porous membrane layers, and hydrogel layers.
[4] The immunoisolation device according to any one of [1] to [3], wherein the immunoisolation membrane suppresses entry of immunoresponsive cells and immune system humoral factors into the embedding chamber.

[5] The immunoisolation device according to any one of [1] to [4], wherein the immunoisolation membrane blocks permeation of immunoresponsive cells and has insulin and glucose permeabilities of 50% or more and an immune system humoral factor permeability of 30% or less.

[6] The immunoisolation device according to [5], wherein the immunoisolation membrane has insulin and glucose permeabilities of 50% or more, and an immune system humoral factor permeability of 10% or less.

[7] The immunoisolation device according to any one of [1] to [6], wherein the immunoisolation membrane has a tensile strength of 1 MPa or more.

[8] The immunoisolation device according to any one of [1] to [7], wherein the immunoisolation membrane comprises a fiber structure layer, and a porous membrane layer and/or a hydrogel layer formed on the fiber structure layer.

[9] The immunoisolation device according to any one of [1] to [7], wherein the immunoisolation membrane comprises a porous membrane layer and a hydrogel layer formed on the porous membrane layer.

[10] The immunoisolation device according to any one of [1] to [8], wherein the immunoisolation membrane is obtainable by applying a polymer raw material to a fiber structure used as a substrate to form a porous membrane among short fibers of the fiber structure.

[11] The immunoisolation device according to any one of [1] to [10], wherein the immunoisolation membrane is obtainable by directly applying a hydrosol solution to a fiber structure used as a substrate and performing hydrogelation by using heat, temperature, light, or chemical action.

[12] The immunoisolation device according to any one of [1] to [9], wherein the immunoisolation membrane is obtainable by directly applying a hydrosol solution to a porous membrane used as a substrate and performing hydrogelation by using heat, temperature, light, or chemical action.

[13] The immunoisolation device according to any one of [1] to [10] and [12], wherein the immunoisolation membrane comprises a porous membrane of an ethylene-vinyl alcohol copolymer.

[14] The immunoisolation device according to any one of [1] to [13], wherein the hydrogel comprises a polyvinyl alcohol-based polymer.

[15] The immunoisolation device according to any one of [1] to [14], wherein the outermost layer surface of the immunoisolation membrane comprises an ethylene-vinyl alcohol copolymer.

[16] The immunoisolation device according to any one of [1] to [15], wherein the outermost layer surface of the immunoisolation membrane is formed of a fiber structure of an ethylene-vinyl alcohol copolymer, and is smoothly compressed.

[17] The immunoisolation device according to any one of [1] to [16], wherein the immunoisolation membrane has a thickness of 10 μm or more and 300 μm or less.

[18] The immunoisolation device according to any one of [1] to [17], wherein the thickness of multiple layers comprising a hydrogel and a porous membrane that form the immunoisolation membrane is 10 μm or more and 300 μm or less.

[19] The immunoisolation device according to any one of [1] to [18], wherein the thickness of multiple layers comprising a fiber structure and a porous membrane that form the immunoisolation membrane is 10 μm or more and 300 μm or less.

[20] The immunoisolation device according to any one of [1] to [19], wherein the thickness of multiple layers comprising a fiber structure and a hydrogel that form the immunoisolation membrane is 10 μm or more and 300 μm or less.

Advantageous Effects of Invention

The present invention provides an immunoisolation device that achieves both a reduction in the diffusion distance, which is effective in improving the permeabilities of materials, such as physiologically active substances and nutrients, and improvement in durability to withstand transplantation for a long period of time.

Brief Description of Drawings

Fig. 1 is a perspective view of a bag-shaped immunoisolation device.
Fig. 2 is a cross-sectional view of a tubular immunoisolation device.
Fig. 3 is a cross-sectional view of an immunoisolation membrane comprising two layers, i.e., a porous membrane and a non-woven fabric.
Fig. 4 is a cross-sectional view of an immunoisolation membrane comprising two layers, i.e., a non-woven fabric and a porous membrane.
Fig. 5 is a cross-sectional view of an immunoisolation membrane in which a non-woven fabric and a porous membrane are integrated.
Fig. 6 is a cross-sectional view of an immunoisolation membrane comprising two layers, i.e., a porous membrane and a hydrogel.

Fig. 7 is cross-sectional view of an immunoisolation membrane obtainable by multiple layer formation of a non-woven fabric and a hydrogel.

Fig. 8 is cross-sectional view of an immunoisolation membrane comprising three layers, i.e., a hydrogel layer, a non-woven fabric layer, and a porous membrane layer.

Fig. 9 is cross-sectional view of an immunoisolation membrane comprising a hydrogel layer and a layer in which a non-woven fabric and a porous membrane are integrated.

Fig. 10 shows an outline of a material permeability test.

Description of Embodiments

In the present specification, the digits subsequent to significant digits should be construed as being rounded off, unless otherwise specified. For example, unless otherwise specified, "1 or less" includes 1.4, and "10 or less" includes 10.4; however, "1 or less" does not include 1.5, and "10 or less" does not include 10.5 or 11.

The immunoisolation device of the present invention comprises an embedding chamber capable of embedding a material to be transplanted and an immunoisolation membrane, and the embedding chamber is covered with the immunoisolation membrane, thereby suppressing the entry of immune cells and cytokines into the embedding chamber. The immunoisolation membrane comprises at least two members selected from the group consisting of fiber structures, porous membranes, and hydrogels.

The "fiber structure" is, for example, a non-woven fabric, a woven fabric, or a knitted fabric, and is preferably a non-woven fabric. Examples of materials of the fiber structure include gelatin, collagen, chitin, chitosan, fibronectin, dextran, cellulose, polyethylene (PE), polypropylene (PP), polyurethane, polyamide, polyester, polyvinyl alcohol (PVA), ethylene-vinyl alcohol copolymers, polylactic acid, polyglycolic acid, polylactic acid-polyglycolic acid copolymers, PVA modified with a monomer such as methacrylic-modified PVA and acrylic-modified PVA, polycaprolactone, polyglycerol sebacate, polyhydroxyalkanoic acid, polybutylene succinate, polymethylene carbonate, cellulose diacetate, cellulose triacetate, methylcellulose, propylcellulose, benzyl cellulose, carboxymethylcellulose, fibroin, silk, and the like. The fiber structure layer may be composed of one fiber structure layer, or two or more fiber structure layers may be laminated to form a single fiber structure layer. When the immunoisolation device of the present invention comprises two or more fiber structure layers, the fiber structure layers may be directly laminated, or a porous membrane layer or a hydrogel layer may be interposed between two fiber structure layers.

The thickness of the fiber structure layer is not particularly limited, and is preferably 10 $\mu$m or more and 290 $\mu$m or less, and more preferably 15 $\mu$m to 150 $\mu$m.

Examples of hydrosols for producing the "hydrogel" include sols that gel in the presence of a metal ion to form hydrogels; sols that gel in response to temperature to form hydrogels; sols that gel in response to pH to form hydrogels; sols that gel in response to light to form hydrogels; and the like. Metal ions and pH are examples of chemical action. To gel these hydrosols, an operation such as bringing a metal ion into contact with a hydrosol, adjusting the temperature to gelling conditions, adjusting the pH to gelling conditions, applying light under gelling conditions, or providing a magnetic field under gelling conditions may be performed depending on the characteristics of the gel used.

Examples of hydrogels obtainable by gelling in the presence of a metal ion include alginate gels obtainable by gelling in the presence of a divalent or trivalent metal ion, preferably an alkaline earth metal ion, such as a calcium ion or a magnesium ion; carrageenan gels obtainable by gelling in the presence of a calcium ion and/or a potassium ion; acrylic acid-based synthesis gels obtainable by gelling in the presence of a sodium ion; and the like.

Examples of temperature-responsive hydrogels include a temperature-responsive hydrogel obtainable by crosslinking poly(N-isopropylacrylamide) with polyethylene glycol (trade name: Mebiol Gel), methylcellulose, hydroxypropyl cellulose, a copolymer of lactic acid and ethylene glycol, a triblock copolymer of polyethylene glycol and polypropylene oxide (trade name: Pluronic (registered trademark), Poloxamer), agarose, polyvinyl alcohol, and the like.

Examples of pH-responsive hydrogels include alginate gels, chitosan gels, carboxymethyl cellulose gels, acrylic acid-based synthesis gels, and the like.

Examples of photoresponsive hydrogels include a synthesis gel in which azobenzene and cyclodextrin are combined in the skeleton, a gel composed of a supramolecule having fumaric acid amide as a spacer, a gel obtainable by crosslinking or bonding via a nitrobenzyl group, a gel composed of modified polyvinyl alcohol, and the like.

The thickness of the hydrogel layer is not particularly limited, and is preferably 10 $\mu$m to 290 $\mu$m, more preferably 20 $\mu$m to 200 $\mu$m, and even more preferably 30 um to 100 $\mu$m.

The hydrogel layer may be composed of one hydrogel layer, or two or more hydrogel layers may be laminated to form a single hydrogel layer. When the immunoisolation device of the present invention comprises two or more hydrogel layers, the hydrogel layers may be directly laminated, or a porous membrane layer or a fiber structure layer may be interposed between two hydrogel layers.

The hydrogel can adjust the permeabilities of, for example, nutritional substances such as glucose, physiologically active substances such as insulin, and immune system humoral factors, the strength, etc. by crosslinking.

The "porous membrane" is a membrane with multiple pores. Whether a membrane is a porous membrane can be confirmed by a scanning electron microscope (SEM) image or a transmission electron microscope (TEM) image of the cross-section of the membrane.

The thickness of the porous membrane is not particularly limited, and is preferably 15 μm to 290 μm, and more preferably 30 μm to 150 μm.

The average pore size of the porous membrane is not particularly limited, and is preferably 0.01 μm to 10 μm, more preferably 0.01 um to 5 μm, and even more preferably 0.01 to 3 μm. The average pore size can be determined by an SEM image or a TEM image.

The maximum pore size of the porous membrane is not particularly limited, and is preferably 0.01 um to 10 μm, more preferably 0.01 um to 5 μm, and even more preferably 0.01 um to 4 um. When the porous membrane has a maximum pore size within the above ranges, the porous membrane can suppress the entry of immunoresponsive cells into the embedding chamber and achieves sufficient permeation of nutritional substances such as amino acids, vitamins, inorganic salts, and carbon sources (such as glucose); oxygen; carbon dioxide; and physiologically active substances such as cytokines, hormones, and insulins. The average pore size or the maximum pore size can be determined from an SEM image or a TEM image.

It is preferred that the porous membrane comprises a polymer and is substantially composed of the polymer. The polymer forming the porous membrane is preferably biocompatible.

The polymer may be, for example, a thermoplastic or thermosetting polymer. The polymer may be biocompatible. Specific examples of polymers include ethylene-vinyl alcohol copolymers, polysulfones, cellulose acetate and like cellulose acylate, nitrocellulose, sulfonated polysulfones, polyethersulfone, polyacrylonitrile, styrene-acrylonitrile copolymers, styrenebutadiene copolymers, saponified products of ethylene-vinyl acetate copolymers, polyvinyl alcohol, polycarbonates, organosiloxane-polycarbonate copolymers, polyester carbonate, organopolysiloxane, polyphenylene oxide, polyamides, polyimides, polyamideimides, polybenzimidazoles, polytetrafluoroethylene (PTFE), and the like. These may be homopolymers, copolymers, polymer blends, polymer alloys, etc. in terms of, for example, solubility, optical properties, electrical properties, strength, and elasticity. Hydrophilic polymers such as polyvinylpyrrolidone, hydroxypropyl cellulose, hydroxyethyl cellulose, and polyethylene glycol may be contained as polymers forming the porous membrane. The biocompatibility can be improved by combining hydrophilic and hydrophobic polymers.

The porous membrane is preferably a membrane formed from one composition as a single layer and preferably does not have a multilayer, laminated structure.

The porous membrane layer may be composed of one porous membrane layer, or two or more porous membrane layers may be laminated to form a single porous membrane layer. When the immunoisolation device of the present invention comprises two or more porous membrane layers, the porous membrane layers may be directly laminated, or a hydrogel layer or a fiber structure layer may be interposed between two porous membrane layers.

The amount of glucose, insulin, an immune system humoral factor, or the like permeating the immunoisolation membrane can be measured by inserting the porous membrane in a connection site between two glass chambers having the same volume, pouring a sample solution of a known concentration of insulin or the like into chamber A under stirring at 37°C, and quantifying the amount of insulin or the like contained in a liquid sampled from chamber B after a certain period of time by ELISA or the like. The amounts of liquids in chambers A and B are adjusted to be equal when the sample solution is poured into chamber A.

The permeability of glucose, insulin, an immune system humoral factor, or the like through the immunoisolation membrane is a value expressed as a percentage of what the amount of each substance permeating into chamber B after 24 hours, measured by the above method, is with respect to the concentration at which equilibrium is reached, i.e., half of the concentration of the substance poured into chamber A. More specifically, for example, the permeability can be determined by the following formula:

$$[\text{permeability (\%) of each substance}]=[\text{concentration of each substance in chamber B after 24 hours of measurement}]/\{[\text{concentration of each substance in chamber A at the start of measurement}]\div 2\}\times 100$$

The immunoisolation membrane of the present invention has insulin and glucose permeabilities of preferably 50% or more, more preferably 90% or more, and even more preferably 95% or more.

The immunoisolation membrane of the present invention has an immune system humoral factor permeability of preferably 30% or less, and more preferably 10% or less.

The immunoisolation membrane of the present invention comprises two or more members selected from the group consisting of fiber structures, hydrogels, and porous membranes, and may contain all three members. The fiber structure, hydrogel, and porous membrane may each form a layer, the layers may be clearly separated at their boundaries, the boundary between two layers may be indistinct, or two or three members may be integrated to form a single layer. For example, the immunoisolation membrane comprising two members, i.e., a fiber structure and a hydrogel, may be a multilayer immunoisolation membrane in which the fiber structure layer and the hydrogel layer are clearly separated, an immunoisolation membrane having a mixed layer of the fiber structure layer and the hydrogel between the fiber structure layer and the hydrogel layer, or an immunoisolation membrane composed of a single layer formed by fully integrating the fiber structure and the hydrogel. The immunoisolation membrane comprising two members, i.e., a fiber structure and a porous membrane, may be a multilayer immunoisolation membrane in which the fiber structure layer and the porous membrane layer are clearly separated, an immunoisolation membrane having a mixed layer of the fiber structure layer and the porous membrane between the fiber structure layer and the porous membrane layer, or an immunoisolation membrane composed of a single layer formed by fully integrating the fiber structure and the porous membrane. The immunoisolation membrane comprising two members, i.e., a hydrogel and a porous membrane, may be a multilayer immunoisolation membrane in which the hydrogel layer and the porous membrane layer are clearly separated, an immunoisolation membrane having a mixed layer of the hydrogel layer and the porous membrane between the hydrogel layer and the porous membrane layer, or an immunoisolation membrane composed of a single layer formed by fully integrating the hydrogel and the porous membrane.

When the immunoisolation membrane comprises multiple layers, the layers may be bonded by an adhesive, heat, pressure, or the like. When adjacent layers are formed of highly compatible materials, the layers can be bonded by sequentially forming the layers. The thickness of the immunoisolation membrane is not particularly limited, and is preferably 10 $\mu$m or more and 300 $\mu$m or less.

The immunoisolation membrane preferably has a tensile strength of 1 MPa or more. The tensile strength can be measured according to JIS K 7127;1999.

The outer layer of the immunoisolation membrane may be formed of a fiber structure, a hydrogel, or a porous membrane, or may be formed of a mixture of two or three of these. The inner layer of the immunoisolation membrane may be formed of a fiber structure, a hydrogel, or a porous membrane, or may be formed of a mixture of two or three of these. The outer layer of the immunoisolation membrane is preferably biocompatible in order to prevent it from being recognized as foreign. The immunoisolation membrane is required to have permeability necessary to allow sufficient oxygen and nutrients to permeate the material to be transplanted inside. Since it is necessary to block the entry of immunocompetent cells into the embedding chamber, it is preferred that the outer surface, inner surface, or inside of the immunoisolation membrane has areas having a pore size that blocks permeation of immunocompetent cells throughout the immunoisolation membrane.

The material to be transplanted may be introduced into the embedding chamber using a device such as syringe for injecting a suspension of the material to be transplanted; or an opening may be provided in the immunoisolation membrane, the material to be transplanted may be introduced into the embedding chamber through the opening, and then the opening may be closed to block the entry of immunoresponsive cells and immune system humoral factors through the opening. Since oxygen and nutrients can permeate through the portion of the immunoisolation membrane other than the opening, the opening can be closed to suppress permeation of substances, including nutrients. After the immunoisolation device of the present invention is implanted in a living body, the material to be transplanted that has reduced function in the embedding chamber may be removed, and a new functional material to be transplanted may be introduced. This operation may be repeated. The immunoisolation device may be used repeatedly for introduction of materials to be transplanted. The immunoisolation device may be removed together with the material to be transplanted. When the immunoisolation device is left in place and only the material to be transplanted is replaced, a tubular access port that is accessible externally may be provided in the immunoisolation device in advance, an end of the access port may be placed externally or subcutaneously, and the material to be transplanted may be replaced through the access port. It is important to join the access port and the device with a highly biocompatible resin, such as an ethylene-vinyl alcohol copolymer, in such a manner as to not cause leakage. It is also important to take measures to prevent infection at the external end by, for example, using a sterilized injection needle or catheter under infection prevention measures and sealing the end with a biocompatible material after the replacement operation.

The immunoisolation membrane of the present invention has sufficient strength, is stable in the body of a recipient, and can suppress the entry of immunocompetent cells into the embedding chamber; thus, the entry of the material to be transplanted that is inside the embedding chamber into the body of a recipient can be also suppressed at the same time. Therefore, even if the material to be transplanted is derived from iPS cells, for which canceration is a concern, the material is safe for use.

In one preferred embodiment of the present invention, a fiber structure with excellent durability is used as a substrate, a porous polymer membrane or a hydrogel, or both a porous membrane and a hydrogel, is formed on the fiber structure, thereby achieving both a reduction in thickness of the device, which improves the diffusion efficiency of a physiologically active substance, and durability due to increased strength, while maintaining the immunoisolation effect.

The immunoisolation device according to a preferred embodiment of the present invention preferably has shape retainability to have sufficient strength in a living body.

Examples of immunoresponsive cells include macrophages, cytotoxic T cells, natural killer cells, dendritic cells, helper T cells, and the like. Examples of immune system humoral factors include antibodies, complements, cytokines, and the like.

The immunoisolation device according to a preferred embodiment of the present invention has a bag shape, a tubular shape, a cylindrical shape, a rectangular cylindrical shape, a spherical shape, a cubical shape, a rectangular-parallelepiped shape, a sheet shape, or a hollow-fiber shape, and cells for transplantation are embedded inside the immunoisolation device. The material to be transplanted may be cells, cell clusters, a cell sheet, or a graft, and physiologically active substances other than cells, such as enzymes, hormones, cytokines, and drugs, can also be used. Preferred cells, cell clusters, and grafts are those that release physiologically active substances out of the immunoisolation device. That is, cells, cell clusters, and grafts contain physiologically active substance-producing cells.

The immunoisolation device according to a particularly preferred embodiment of the present invention comprises any of the following four multilayer structures (i) to (iv):

(i) a fiber structure or a hydrogel is used as a substrate, and a porous membrane is formed on the substrate;
(ii) a fiber structure is used as a substrate, and a hydrogel is formed by impregnating the hydrogel into the fiber structure;
(iii) a porous membrane is used as a substrate, and a hydrogel is impregnated onto the porous membrane;
(iv) a fiber structure is used as a substrate, a porous membrane is formed, and further, a hydrogel is formed.

To maintain the functionality of the device, the fiber structure, porous membrane, and hydrogel are preferably formed of materials that are excellent in safety and biocompatibility.

The material permeability can be controlled by the pore size of the porous membrane or the strength and degree of crosslinking of the hydrogel. To exhibit an immunoisolation function, it is desirable that the pore size of the porous membrane is less than or equal to a size that does not allow cells to pass therethrough and that the hydrogel can suppress the permeation of immune response factors such as cells and antibodies without suppressing the permeation of physiologically active substances.

To improve durability, it is desirable that high-strength fibers are used for the fiber structure.

To prevent adhesion to surrounding tissue and fibrosis, it is desirable that the immunoisolation membrane is composed of a material with excellent biocompatibility. When the immunoisolation membrane is composed of multiple materials, it is desirable that a material with excellent biocompatibility is used as the transplantation side, i.e., the contact surface of the outermost layer that comes into contact with the transplantation site in a recipient. Examples of materials with excellent biocompatibility include ethylene-vinyl alcohol copolymers.

Preferred embodiments of the present invention are described below with reference to the drawings.

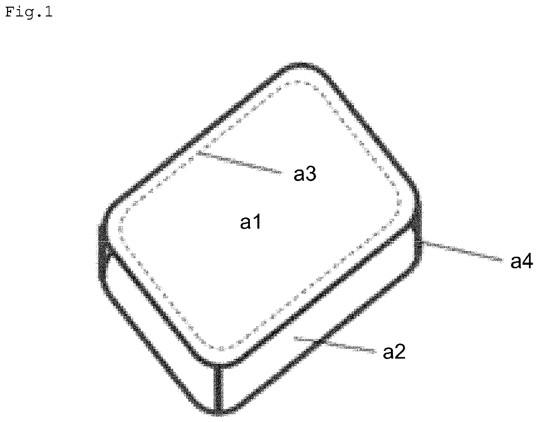

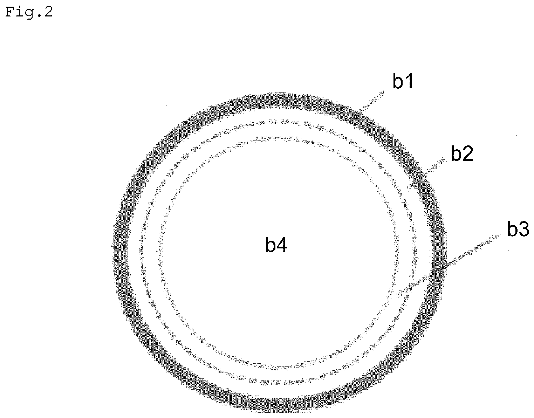

As device schematic views, Fig. 1 shows an immunoisolation membrane molded into a bag, and Fig. 2 shows an immunoisolation membrane molded into a tube. The bag-shaped device (Fig. 1) is molded by fusing (a3) multiple immunoisolation membranes (a1 and a2) as shown below by using heat, ultrasound, high frequency, or electron beam while having a certain distance (a4) to ensure an embedding chamber space. A spacer may be provided to ensure a certain distance (a4).

The tubular device (Fig. 2) is formed of immunoisolation membranes (b1, b2, and b3) molded into a tubular shape. The tubular device is molded by allowing the embedding chamber to be embedded in the tubular interior (b4), and fusing and sealing both of the tubular ends by heat, ultrasound, high frequency, electron beam, or the like.

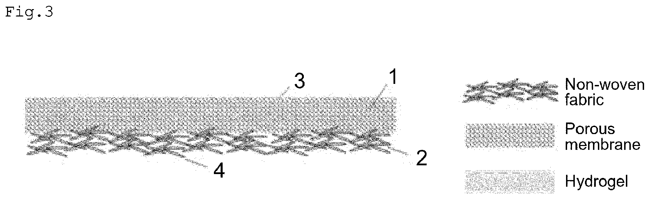

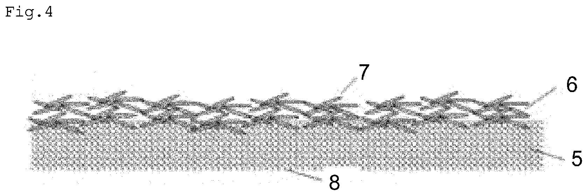

In Figs 3 to 9, the outermost layer is in contact with the transplantation site, and the innermost layer is in contact with the embedding chamber. Figs. 3 and 4 show the multiple layer formation of a porous membrane (1) and a fiber structure (2).

Fig. 3 shows a two-layer structure in which the porous membrane (1) is the outer layer and the fiber structure (2) is the inner layer, and the outermost layer (3) is in contact with the site to be transplanted and the innermost layer (4) is in contact with the embedding chamber.

Fig. 4 shows a two-layer structure in which the fiber structure (6) is the outer layer and the porous membrane (5) is the inner layer, and the outermost layer (7) is in contact with the site to be transplanted and the innermost layer (8) is in contact with the embedding chamber.

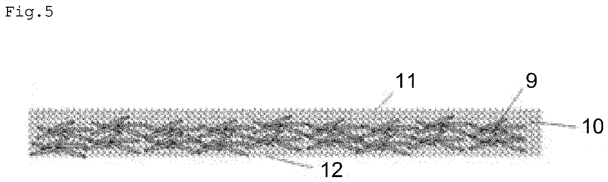

Fig. 5 shows a composite layer obtainable by directly applying the porous membrane (10) to the substrate of the fiber structure (9).

In the outermost layer surface (11), the porous membrane (10) covers the fiber structure (9), and the innermost layer surface (12) may be either the porous membrane (10) or the fiber structure (9).

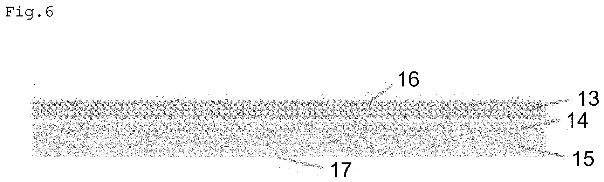

Fig. 6 shows the multiple layer formation of the porous membrane (13) and the hydrogel (15). The hydrogel (15) is impregnated and fixed (14) in the porous membrane (13), the outermost layer surface (16) is formed of a porous membrane (10), and the innermost layer surface is formed of a hydrogel (17).

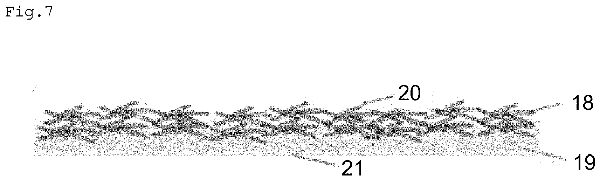

Fig. 7 shows the multiple layer formation of the fiber structure (18) and the hydrogel (19). The hydrogel (19) is impregnated and fixed in the fiber structure (18), the outermost layer surface (20) is formed of a fiber structure (18), and the innermost layer surface (21) is formed of a hydrogel (19).

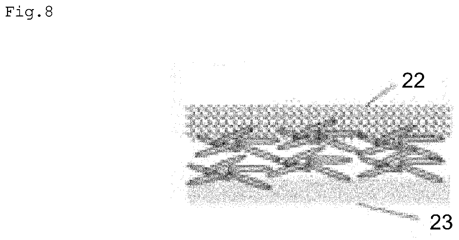

Fig. 8 shows a composite layer obtainable by composing the multilayer devices of Figs. 3 and 7, wherein the outermost layer surface (22) is formed of a porous membrane and the innermost layer surface (23) is formed of a hydrogel.

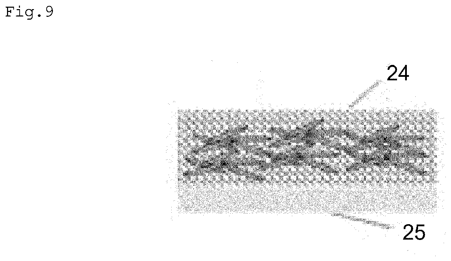

Fig. 9 shows a composite layer obtainable by composing the multilayer devices of Figs. 5 and 6, wherein the outermost layer surface (24) is formed of a porous membrane and the innermost layer surface (25) is formed of a hydrogel.

The present invention relates to a device for transplantation for use in cell transplantation therapy and the like, and particularly to an immunoisolation device for protection against immune rejection reaction of a material to be transplanted.

The immunoisolation device is intended to be mainly used for cell transplantation therapy as a regenerative medicine product; however, it can also be applied to the transplantation of physiologically active substances other than cells, such as enzymes, hormones, and drugs.

As shown in Fig. 1 or Fig. 2, the device concept view shows that the device has a bag or tubular shape. In the device, a material to be transplanted, which is to be transplanted, such as cells or cell clusters, is embedded as an embedding chamber. The embedding chamber indicates a chamber in which the material to be transplanted, such as cells or cell clusters, is uniformly fixed in a dispersed state in the fixation material inside the embedding chamber. The fixation material may be a hydrogel or hydrosol as described above, or a cell scaffold material such as collagen fibers. As the fixation material, a hydrosol, a hydrogel, or a cell scaffold material that is subjected to sterilization treatment can be used. The fixation material may be present before the introduction of the material to be transplanted into the embedding chamber surrounded by the immunoisolation membrane, and the material to be transplanted and the fixation material may be introduced later into the immunoisolation device formed of an immunoisolation membrane.

When the immunoisolation membrane of the present invention is a single porous membrane or a combination of a porous membrane and a fiber structure, it is preferable to combine a hydrogel as a fixing material. This is because the hydrogel can effectively suppress the contact between immune system humoral factors and a material to be transplanted. On the other hand, if the immunoisolation membrane comprises multiple layers (two or three layers) of a combination of a porous membrane and/or a fiber structure with a hydrogel, the hydrogel in the immunoisolation membrane suppresses the entry of immune system humoral factors. Accordingly, the fixation material may be a hydrogel, or a scaffold material other than a hydrogel that is capable of dispersing and fixing a material to be transplanted can be widely used.

In addition to the role of uniformly fixing cells by adjusting a fixation material such as a hydrogel, the embedding chamber can have a role of preventing the entry of immune system humoral factors such as antibodies into the material to be transplanted.

The immunoisolation membranes shown in Figs. 3 to 9 are composed of a combination of several materials. In Figs. 3 to 9, the outermost layer is in contact with a recipient's transplant site tissue, and the innermost layer is in contact with the embedding chamber. These immunoisolation membranes are used to form a bag (Fig. 1) or tubular shape (Fig. 2); and an embedding chamber, in which a cell or cell mass, which is a material to be transplanted is fixed therein is embedded, thus obtaining an immunoisolation device.

Figs. 3 and 4 show the multiple layer formation of the porous membrane (1 or 5) and the fiber structure (2 or 6). Figs. 3 and 4 show an immunoisolation membrane obtainable by applying the porous membrane layer on the fiber structure layer. The thickness of multiple layers comprising the fiber structure and porous membrane that form the immunoisolation membrane is not particularly limited, and is preferably 10 um or more and 300 $\mu$m or less.

Fig. 3 shows a two-layer structure in which the porous membrane (1) is the outer layer and the fiber structure (2) is the inner layer, and the outermost layer (3) is in contact with the site to be transplanted and the innermost layer (4) is in contact with the embedding chamber.

Fig. 4 shows a two-layer structure in which the fiber structure (6) is the outer layer and the porous membrane (5) is the inner layer, and the outermost layer (7) is in contact with the site to be transplanted and the innermost layer (8) is in contact with the embedding chamber.

It is desirable that the porous membrane has a pore size of 5 um or less, which is smaller than the cell size, due to the need to suppress cell permeation from the recipient and the need to have a function of preventing leakage of cells of a material to be transplanted. As a membrane material, materials with excellent biocompatibility that are less likely to cause adhesion or inflammation with the recipient's transplantation-surrounding tissues are desirable. For example, ethylene-vinyl alcohol copolymers and cellulose are desirable.

It is desirable that the film thickness of the multilayer membrane is as thin as possible, not more than 100 $\mu$m, considering the mass diffusion efficiency of the physiologically active substance from the material to be transplanted.

The fiber structure is obtainable by thermally, mechanically, or chemically bonding fibers, or by combining fibers. By adjusting the basis weight (basis weight amount) according to the fiber diameter or amount, the permeability and filtration properties can be adjusted in addition to the strength. For example, the basis weight (basis weight amount) is preferably 10 to 200 $g/m^2$, and more preferably 50 to 150 $g/m^2$. The basis weight can be measured by cutting out a fiber structure of a given area and measuring the weight. The fiber structure preferably has an average pore size of 1 to 100 $\mu$m, and more preferably 1 to 50 $\mu$m in terms of retention properties of a cell mass, which is a material to be transplanted. The average pore size can be measured by the method described in JIS K 3832-1990 (test solution: distilled water; test gas: air; method of moistening the test filter: method B). Figs. 1 and 2 each show a concept view in which the porous membrane, which is the outer layer, and the fiber structure are thermally, mechanically, or chemically bonded or fixed to form multiple layers. It is desirable that the thickness of the fiber structure is as thin as possible, not more than 100 $\mu$m, considering the mass diffusion efficiency of the physiologically active substance from the material to be transplanted. However, it is necessary to fully consider that the main object of the multiple layer formation of the fiber structure is to improve the durability of the immunoisolation membrane containing the porous membrane.

As in the porous membrane, a material having excellent biocompatibility is desirable as the fiber material of the fiber structure. However, in the case of Fig. 3, since the membrane with which the recipient is in contact is the porous membrane, which is the outermost layer, the fiber material is not limited to the above as long as the material does not adversely affect a material to be transplanted. In contrast, in Fig. 4, since the outermost layer is the fiber structure, it is desirable that the fiber material of the fiber structure is a material having high biocompatibility. It is desirable that the surface of the fiber structure is smoothed by thermal, mechanical, or chemical treatment. The material having excellent biocompatibility include ethylene-vinyl alcohol copolymers.

Fig. 5 shows a composite layer obtainable by directly applying the porous membrane (10) to the substrate of the fiber structure (9). The difference between Fig. 5, and Figs. 3 and 4 is that Fig. 5 does not show a two-layer structure in which the fiber structure and the porous membrane are separately produced and bonded together; rather, it shows a one-layer structure in which the porous membrane is present in the fiber structure of the fiber structure. For example, the porous membrane can be formed among short fibers of the fiber structure by applying a polymer solution on the substrate of the fiber structure, and solidifying the polymer raw material by phase separation, which is a phase transition phenomenon.

Specifically, the solution of a polymer constituting the porous membrane is applied, and is impregnated into the fiber structure as a substrate; then, the resultant is exposed to a poor solvent in which the polymer is insoluble or a low-temperature environment or the like, thereby precipitating and solidifying the polymer. Thus, the porous membrane layer is applied on the surface of the fiber structure or in the fiber structure.

From the above, the thickness of the porous membrane is determined by the fiber thickness of the fiber structure. The thickness of the immunoisolation membrane can be reduced as compared to those of Figs. 3 and 4, while at the same time, the strength of the porous membrane can be improved depending on the strength of the fiber structure.

In the outermost layer surface (11), the porous membrane (10) covers the fiber structure (9), and the innermost layer surface (12) may be either the porous membrane (10) or the fiber structure (9). As in the case of the porous membrane, it is desirable that the fiber material of the fiber structure is a material having excellent biocompatibility. Since the membrane with which the recipient is in contact is the porous membrane, which is the outermost layer, it is desirable that the porous membrane is a material having excellent biocompatibility; however, the fiber material of the fiber structure is not limited thereto as long as the fiber structure does not adversely affect the material to be transplanted.

Fig. 6 shows the multiple layer formation of the porous membrane (13) and the hydrogel (15). The hydrogel (15) is impregnated and fixed (14) in the porous membrane (13), the outermost layer surface (16) is formed of the porous membrane (10), and the innermost layer surface is formed of the hydrogel (17). Fig. 6 shows an immunoisolation membrane in which the hydrogel layer is formed on the porous membrane layer. The thickness of multiple layers comprising the hydrogel and the porous membrane that form the immunoisolation membrane is not particularly limited, and is preferably 10 $\mu$m or more and 300 $\mu$m or less.

It is desirable that the porous membrane has a pore size of 5 um or less, which is smaller than the cell size, due to the need to have a function of suppressing cell invasion from the recipient and preventing leakage of cells which is a material to be transplanted. As a membrane material, a material having excellent biocompatibility that is less likely to cause adhesion or inflammation with the transplantation-surrounding tissues of a recipient is desirable. For example, ethylene-vinyl alcohol copolymers and cellulose are desirable.

Although cell invasion and cell leakage can be suppressed in the porous membrane, it is not easy to suppress the invasion of immune system humoral factors such as IgG antibodies without suppressing the permeability of necessary physiologically active substances. Therefore, by adjusting the gel strength or density of crosslinking of the hydrogel impregnated and fixed in the porous membrane, it is possible to suppress the invasion of immune system humoral factors such as IgG antibodies without suppressing the permeability of physiologically active substances. Preferable examples of the hydrogel include polyvinyl alcohol-based polymers, chitosan, and alginic acid salts.

Multiple layer formation is performed by directly applying a hydrosol solution to a porous membrane, which is used as a substrate, and performing hydrogelation by using heat, temperature, light, or chemical action.

The porous membrane can be formed among short fibers of the fiber structure by directly applying the polymer solution of the porous membrane using a hydrogel as a substrate, and solidifying the polymer raw material by phase separation, which is a phase transition phenomenon. Some porous membrane raw materials may require pre-treatment to reduce the water content by pre-drying the hydrogel.

It is desirable that the porous membrane, which is the outer layer, is a material having more excellent biocompatibility than that of the hydrogel, and serves to protect the hydrogel from causing adhesion or inflammation with the recipient's transplantation-surrounding tissues.

Fig. 7 shows the multiple layer formation of the fiber structure (18) and the hydrogel (19). The hydrogel (19) is impregnated and fixed in the fiber structure (18), the outermost layer surface (20) is formed of the fiber structure (18), and the innermost layer surface (21) is formed of the hydrogel (19). Fig. 7 shows an immunoisolation membrane in which the hydrogel layer is formed on the fiber structure. The thickness of multiple layers comprising the fiber structure and the hydrogel that form the immunoisolation membrane is not particularly limited, and is preferably 10 μm or more and 300 μm or less.

In the single fiber structure, it is not easy to suppress the invasion of immune system humoral factors such as IgG antibodies in addition to cell invasion and cell leakage, without suppressing the permeability of necessary physiologically active substances. Thus, by adjusting the gel strength or density of crosslinking of the hydrogel impregnated and fixed in the fiber structure, the invasion of immune system humoral factors such as IgG antibodies can be suppressed in addition to the cell invasion and cell leakage, without suppressing the permeability of physiologically active substances. Preferable examples of the hydrogel include polyvinyl alcohol-based polymers, chitosan, and alginic acid salts.

The polyvinyl alcohol-based polymer can be produced, for example, by saponification of a polyvinyl ester obtainable by polymerizing a vinyl ester-based monomer, and converting the ester group in the polyvinyl ester to a hydroxyl group.

Examples of the vinyl ester-based monomer include vinyl formate, vinyl acetate, vinyl propionate, vinyl n-butyrate, vinyl isobutyrate, vinyl pivalate, vinyl versatate, vinyl caproate, vinyl caprylate, vinyl caprate, vinyl laurate, vinyl myristate, vinyl palmitate, vinyl stearate, vinyl oleate, and like aliphatic vinyl esters; vinyl benzoate, and like aromatic vinyl esters. These vinyl ester-based monomers may be used alone or in a combination of two or more.

Among the vinyl ester-based monomers, aliphatic vinyl esters are preferred, and vinyl acetate is more preferred in view of production cost. In other words, the polyvinyl ester is preferably polyvinyl acetate obtainable by polymerization of vinyl acetate.

The polyvinyl ester may contain a structural unit derived from other monomers than vinyl ester monomers, as required, to the extent that the effect of the present invention is not impaired. Examples of other monomers include α-olefins such as ethylene, propylene, n-butene, and isobutylene; acrylic acid or salts thereof; alkyl acrylates such as methyl acrylate, ethyl acrylate, n-propyl acrylate, i-propyl acrylate, n-butyl acrylate, i-butyl acrylate, t-butyl acrylate, 2-ethylhexyl acrylate, dodecyl acrylate, and octadecyl acrylate; methacrylic acid or salts thereof; alkyl methacrylates such as methyl methacrylate, ethyl methacrylate, n-propyl methacrylate, i-propyl methacrylate, n-butyl methacrylate, i-butyl methacrylate, t-butyl methacrylate, 2-ethylhexyl methacrylate, dodecyl methacrylate, and octadecyl methacrylate; acrylamide derivatives such as acrylamide, N-methylacrylamide, N-ethylacrylamide, N,N-dimethylacrylamide, diacetoneacrylamide, acrylamidopropane sulfonic acid or salts thereof, acrylamidopropyl dimethylamine or salts or quaternary salts thereof, N-methylolacrylamide or derivatives thereof; methacrylamide derivatives such as methacrylamide, N-methylmethacrylamide, N-ethylmethacrylamide, methacrylamidopropanesulfonic acid or salts thereof, methacrylamidopropyl dimethylamine or salts or quaternary salts thereof, N-methylolmethacrylamide or derivatives thereof; N-vinylamide derivatives such as N-vinylformamide and N-vinylacetamide; vinyl ethers such as methyl vinyl ether, ethyl vinyl ether, n-propyl vinyl ether, i-propyl vinyl ether, n-butyl vinyl ether, i-butyl vinyl ether, t-butyl vinyl ether, dodecyl vinyl ether, and stearyl vinyl ether; nitriles such as acrylonitrile and methacrylonitrile; vinyl halides such as vinyl chloride and vinyl fluoride; vinylidene halides such as vinylidene chloride and vinylidene fluoride; allyl compounds such as allyl acetate and allyl chloride; maleic acid or salts thereof, ester or acid anhydride; vinyl silyl compounds such as vinyl trimethoxysilane; isopropenyl acetate; etc. These can be used alone or in a combination of two or more.

The average polymerization degree of the polyvinyl alcohol-based polymer in the present specification is the average polymerization degree measured according to JIS K 6726:1994, as described above. Specifically, the average polymerization degree can be determined from the intrinsic viscosity that is measured in water at 30°C after raw material PVA has been saponified and purified.

The degree of saponification of the polyvinyl alcohol-based polymer is preferably 50 mol% or more, more preferably 60 mol% or more, and even more preferably 65 mol% or more, from the viewpoint of improving the water solubility of the polyvinyl alcohol-based polymer.

From the viewpoint of inhibiting an uncured gel solution described below from having high viscosity, and improving the storage stability of the uncured gel solution, the degree of saponification of the polyvinyl alcohol-based polymer is preferably 99 mol% or less.

In the present specification, the saponification degree of a polyvinyl alcohol-based polymer means the ratio (mol%) of the number of moles of the vinyl alcohol unit to the total number of moles of the structural unit (e.g., vinyl acetate unit) that can be converted to a vinyl alcohol unit by saponification in the raw material PVA and a vinyl alcohol unit. The saponification degree of a polyvinyl alcohol-based polymer can be measured according to JIS K 6726:1994.

By the etherification reaction of a compound containing an ethylenically unsaturated group and a glycidyl group in the molecule (e.g., (meth)acrylic acid glycidyl, allyl glycidyl ether, etc.) with the hydroxyl group of the side chain of the polyvinyl alcohol-based polymer in the presence of a base, a (meth)acryloyl group and/or an allyl group can be introduced to the polyvinyl alcohol-based polymer.

Multiple layer formation is performed by directly applying a hydrosol solution to a fiber structure, which is used as a substrate, and performing hydrogelation by using heat, temperature, light, or chemical action.

It is desirable that the fiber structure, which is the outer layer, is a material having more excellent biocompatibility than that of the hydrogel, and examples include ethylene-vinyl alcohol copolymers. Since the fiber structure also serves to protect the hydrogel from causing adhesion or inflammation with the recipient's tissues, it is desirable that the surface of the fiber structure, which is the outermost layer, is smoothed by thermal, mechanical, or chemical treatment.

Fig. 8 shows a composite layer obtainable by composing the multilayer devices of Figs. 3 and 7, wherein the outermost layer surface (22) is formed of a porous membrane and the innermost layer surface (23) is formed of a hydrogel.

Multiple layer formation is performed by applying the hydrosol solution to the fiber structure site of multiple layers comprising a porous membrane and a fiber structure, and performing hydrogelation by using heat, temperature, light, or chemical action.

The three-layer structure provides stronger immunoisolation properties than those of the two-layer structure, and also improves the strength as an immunoisolation membrane. Both the permeability and immunoisolation properties can be adjusted by the pore size of the porous membrane, which is the outer layer, and the gel strength and degree of crosslinking of the hydrogel, which is the inner layer.

Fig. 9 shows a composite layer obtainable by composing the multilayer devices of Figs. 5 and 6, wherein the outermost layer surface (24) is formed of a porous membrane and the innermost layer surface (25) is formed of a hydrogel.

The multiple layer formation with a hydrogel provides stronger immunoisolation properties and also improves the strength as an immunoisolation membrane. Both the permeability and immunoisolation can be adjusted by the pore size of the porous membrane, which is the outer layer, and the gel strength and degree of crosslinking of the hydrogel, which is the inner layer.

Examples

The invention is described in more detail below on the basis of examples.

Example 1: Multiple layer formation by bonding

1) Multiple layer formation

Multiple layer formation of a porous membrane (average pore size 1.8 $\mu$m, maximum pore size 3.4 $\mu$m) formed by a polymer phase separation reaction using an ethylene-vinyl alcohol copolymer, and a non-woven fabric (weight basis: 51.5 g/m$^2$, average pore size: 20 $\mu$m) produced by a melt-blown method using an ethylene-vinyl alcohol copolymer was performed.

For the multiple layer formation, the porous membrane was layered on a material in which a mist of dimethyl sulfoxide containing 5 wt% of ion exchange water was sprayed on the joining site of the porous membrane and the non-woven fabric. A pressure of 200 kg/cm$^2$ was applied from the side of the non-woven fabric to conduct pressure-bonding under heating at 60°C.

A 100-pm-thick porous membrane and a 200-$\mu$m-thick non-woven fabric were used for trial production. The tensile strength (JIS K 7127; 1999) of the porous membrane and the non-woven fabric was respectively 1.5 MPa and 5 MPa. By the multiple layer formation, the tensile strength was increased to 5 MPa from 1.5 MPa, which was the tensile strength of the single porous membrane.

Measurement conditions
Name of device: Model 210XL universal testing machine (Intesco Co., Ltd.)
Test speed: 50 mm/min
Sample shape: type 5
Test temperature: 23.0°C

2) Material permeability test

The glucose, insulin, and IgG permeabilities for such a porous membrane and non-woven fabric were measured. From each permeability, the estimated value of the permeability in the case of multiple layer formation was calculated. The estimated value is based on the assumption that the pores of the porous membrane do not collapse at the joining interface due to the multiple layer formation.

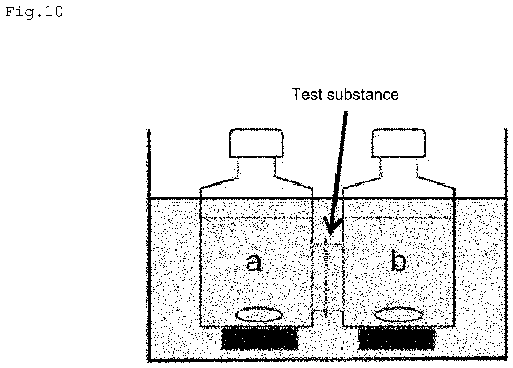

The measurement was conducted by the following method. The sample was placed between chambers a and b (Fig. 10); insulin (30 U/L), glucose (5 mg/mL), and IgG (0.5 pg/mL) were prepared in chamber a, and then stirring was performed with a stirrer under constant temperature at 37°C. 24 hours later, the concentration changes of insulin, glucose, and IgG in chamber b were measured by ELISA, thus calculating the permeability of each material (Table 1).

Table 1

| Permeability after 24 hours | Porous membrane alone | Non-woven fabric alone | Multiple layer formation (Estimated value) |
| --- | --- | --- | --- |
| Glucose | 100% | 100% | 100% |
| Insulin | 84.8% | 100% | 80.6% |
| IgG | 5.2% | 19.70 | 4.2% |

A bag-shaped device formed of a multilayer membrane and a porous membrane in which mouse fibroblast NIH/3T3 cells ($2*10^6$ cells) were encapsulated was prepared, and cultured in the wells of a 6-well plate. The culture surface was observed on day 7, and cells attached to the wells and a culture supernatant were collected to count cell nuclei. The results indicate that no cell invasion was observed as in the case of the single porous membrane.

Example 2: Multiple layer formation by impregnation

A non-woven fabric produced from polypropylene fiber by a melt-blown method was used as a substrate, and a porous membrane was formed on the non-woven fabric by a polymer phase separation reaction, using an ethylene-vinyl alcohol copolymer.

A 200-$\mu$m-thick polypropylene non-woven fabric was used for trial production. The tensile strength of the non-woven fabric was 5 MPa.

For multiple layer formation, the non-woven fabric was immersed in a dimethyl sulfoxide solution of 20 wt% ethylene-vinyl alcohol copolymer heated to 80°C for 90 seconds, and then immersed in a coagulated liquid DMSO/water 50% at 40°C for 15 minutes. Thus, a porous membrane was obtained by a phase separation reaction.

By the multiple layer formation by impregnation, the membrane thickness was 200 $\mu$m, which was the same as the thickness of the single non-woven fabric, and the tensile strength was increased to 5 MPa from 1.5 MPa, which was the tensile strength of the single porous membrane. The average pore size was 0.1 $\mu$m and the maximum pore size was 1.5 $\mu$m.

Example 3 (membrane + PVA hydrogel)

1) Multiple layer formation

Multiple layer formation of a porous membrane obtained by a polymer phase separation reaction using an ethylene-vinyl alcohol copolymer and a hydrogel mainly comprising a polyvinyl alcohol was performed.

A 100-$\mu$m-thick porous membrane was used for trial production.

For the multiple layer formation, a sol obtained by adding a phenyl (2,4,6-trimethylbenzoyl) phosphinate lithium salt, which was a water-soluble photoradical polymerization initiator, to 10% of a methacryloyl-modified polyvinyl alcohol (average polymerisation degree: 1700, saponification degree: 98.0 to 99.0 mol%, methacryloyl group denaturation rate: 1.2 mol%) to have a concentration of 0.1% by mass, and dissolving was used. The sol was applied onto a PET film to a thickness of 200 $\mu$m using a bar coater, and then a porous membrane was placed. The sol and the porous membrane were firmly adhered using a laminator. Thereafter, the sol surface was placed on top, and irradiation was performed with 365 nm light at an intensity of 15 mW/cm$^2$ for 3 minutes, thus forming the hydrogel on the porous membrane.

2) Material permeability test

For multilayer membranes, the glucose, insulin, and IgG permeabilities were measured. The sample was placed between chambers a and b (Fig. 10); insulin (30 U/L), glucose (5 mg/mL), and IgG (0.5 pg/mL) were prepared in chamber a, and then stirring was performed with a stirrer under constant temperature at 37°C. 24 hours later, the concentration changes of insulin, glucose, and IgG in chamber b were measured by ELISA, thus calculating the permeability of each material. The results indicate that the glucose, insulin, and IgG permeabilities were respectively 93.4%, 62.8%, and 2.5% (Table 2). A bag-shaped device in which mouse fibroblast NIH/3T3 cells ($2*10^6$ cells) were encapsulated in the multilayer membrane was prepared, and cultured in the wells of a 6-well plate. The culture surface was observed on day 7, and cells attached to the wells and a culture supernatant were collected to count cell nuclei. The results indicate that no cell invasion was observed as in the case of the single porous membrane.

In conventional devices in which a material to be transplanted is encapsulated in a hydrogel, the thickness of the hydrogel to completely encapsulate a material to be transplanted. For example, a thickness of about 500 μm is needed to encapsulate a cell mass having a diameter of 150 um. A hydrogel having a thickness of 500 μm was prepared using a polyvinyl alcohol modified with a methacryloyl group, and a substance permeability test was performed. The glucose and insulin permeabilities were respectively 54% and 6.8%. This confirmed that the composite membrane of a thin-layer PVA gel exhibited higher mass diffusion efficiency than conventional devices.

Table 2

| Permeability after 24 hours | Porous membrane alone | Multiple layer formation |
|---|---|---|
| Glucose | 100% | 93.4% |
| Insulin | 84.8% | 62.9% |
| IgG | 5.2% | 2.5% |

Example 4 (Non-woven fabric + PVA hydrogel)

Multiple layer formation of a non-woven fabric produced using an ethylene-vinyl alcohol copolymer by a melt-blown method and a hydrogel mainly comprising a polyvinyl alcohol was performed.

A 200-μm-thick ethylene-vinyl alcohol copolymer non-woven fabric was used for trial production. The tensile strength of the non-woven fabric was 5 MPa.

For multiple layer formation, the methacryloyl-modified polyvinyl alcohol solution as described in Example 3 was used. A hydrogel was formed on the membrane of the non-woven fabric in the same manner as in Example 3.

The glucose, insulin, and IgG permeabilities of the multilayer membrane were measured in the same manner as in Example 3. The glucose and insulin permeabilities were respectively 95.3% and 74.3%. The IgG permeability in the single non-woven fabric was 19.70; however, it could be reduced to 7.8% when multiple layers of non-woven fabric and hydrogel ere formed (Table 3).

Furthermore, a bag-shaped device in which mouse fibroblast NIH/3T3 cells ($2*10^6$ cells) were encapsulated in the multilayer membrane was prepared, and cultured in the wells of a 6-well plate. The culture surface was observed on day 7, and cells attached to the wells and a culture supernatant were collected to count cell nuclei. The results indicate that no cell invasion was observed as in the case of the single porous membrane.

Table 3

| Permeability after 24 hours | Non-woven fabric alone | Multiple layer formation |
|---|---|---|
| Glucose | 100% | 95.3% |
| Insulin | 100% | 74.3% |
| IgG | 19.70 | 7.8% |

Example 5 (multilayer membrane obtained by impregnation + PVA hydrogel)

Multiple layer formation of the multilayer membrane produced in Example 2 and a hydrogel mainly comprising a polyvinyl alcohol can be performed. For multiple layer formation, using the same method as in Example 3, the hydrogel can be formed on the multilayer membrane produced in Example 2.

Explanation of Reference Symbols

a1 Immunoisolation membrane
a2 Immunoisolation membrane
a3 Fusing
a4 Certain distance
b1 Immunoisolation membrane
b2 Immunoisolation membrane
b3 Immunoisolation membrane
b4 Tubular interior
1 Porous membrane
2 Fiber structure
3 Outermost layer
4 Innermost layer
5 Porous membrane
6 Fiber structure
7 Outermost layer
8 Innermost layer
9 Fiber structure
10 Porous membrane
11 Outermost layer surface
12 Innermost layer surface
13 Porous membrane
14 Impregnation and fixation
15 Hydrogel
16 Outermost layer surface
17 Hydrogel
18 Fiber structure
19 Hydrogel
20 Outermost layer surface
21 Innermost layer surface
22 Outermost layer surface
23 Innermost layer surface
24 Outermost layer surface
25 Innermost layer surface

**Claims**

1. An immunoisolation device comprising an embedding chamber for a material to be transplanted, the embedding chamber being covered with an immunoisolation membrane.

2. The immunoisolation device according to claim 1, wherein the immunoisolation membrane comprises at least two members selected from the group consisting of fiber structures, porous membranes, and hydrogels.

3. The immunoisolation device according to claim 1, wherein the immunoisolation membrane is a multilayer membrane comprising at least two layers selected from the group consisting of fiber structure layers, porous membrane layers, and hydrogel layers.

4. The immunoisolation device according to any one of claims 1 to 3, wherein the immunoisolation membrane suppresses entry of immunoresponsive cells and immune system humoral factors into the embedding chamber.

5. The immunoisolation device according to any one of claims 1 to 4, wherein the immunoisolation membrane blocks permeation of immunoresponsive cells and has insulin and glucose permeabilities of 50% or more and an immune system humoral factor permeability of 30% or less.

6. The immunoisolation device according to claim 5, wherein the immunoisolation membrane has insulin and glucose permeabilities of 50% or more, and an immune system humoral factor permeability of 10% or less.

7. The immunoisolation device according to any one of claims 1 to 6, wherein the immunoisolation membrane has a tensile strength of 1 MPa or more.

8. The immunoisolation device according to any one of claims 1 to 7, wherein the immunoisolation membrane comprises a fiber structure layer, and a porous membrane layer and/or a hydrogel layer formed on the fiber structure layer.

9. The immunoisolation device according to any one of claims 1 to 7, wherein the immunoisolation membrane comprises a porous membrane layer and a hydrogel layer formed on the porous membrane layer.

10. The immunoisolation device according to any one of claims 1 to 8, wherein the immunoisolation membrane is obtainable by applying a polymer raw material to a fiber structure used as a substrate to form a porous membrane among short fibers of the fiber structure.

11. The immunoisolation device according to any one of claims 1 to 10, wherein the immunoisolation membrane is obtainable by directly applying a hydrosol solution to a fiber structure used as a substrate and performing hydrogelation by using heat, temperature, light, or chemical action.

12. The immunoisolation device according to any one of claims 1 to 9, wherein the immunoisolation membrane is obtainable by directly applying a hydrosol solution to a porous membrane used as a substrate and performing hydrogelation by using heat, temperature, light, or chemical action.

13. The immunoisolation device according to any one of claims 1 to 10 and 12, wherein the immunoisolation membrane comprises a porous membrane of an ethylene-vinyl alcohol copolymer.

14. The immunoisolation device according to any one of claims 1 to 13, wherein the hydrogel comprises a polyvinyl alcohol-based polymer.

15. The immunoisolation device according to any one of claims 1 to 14, wherein the outermost layer surface of the immunoisolation membrane comprises an ethylene-vinyl alcohol copolymer.

16. The immunoisolation device according to any one of claims 1 to 15, wherein the outermost layer surface of the immunoisolation membrane is formed of a fiber structure of an ethylene-vinyl alcohol copolymer, and is smoothly compressed.

17. The immunoisolation device according to any one of claims 1 to 16, wherein the immunoisolation membrane has a thickness of 10 $\mu$m or more and 300 $\mu$m or less.

18. The immunoisolation device according to any one of claims 1 to 17, wherein the thickness of multiple layers comprising a hydrogel and a porous membrane that form the immunoisolation membrane is 10 $\mu$m or more and 300 $\mu$m or less.

19. The immunoisolation device according to any one of claims 1 to 18, wherein the thickness of multiple layers comprising a fiber structure and a porous membrane that form the immunoisolation membrane is 10 $\mu$m or more and 300 $\mu$m or less.

20. The immunoisolation device according to any one of claims 1 to 19, wherein the thickness of multiple layers comprising a fiber structure and a hydrogel that form the immunoisolation membrane is 10 $\mu$m or more and 300 $\mu$m or less.

Fig.1

Fig.2

b1

b2

b3

b4

Fig.3

3      1

2

4

Non-woven fabric

Porous membrane

Hydrogel

Fig.4

Fig.5

Fig.6

Fig.7

Fig.8

Fig.9

24

25

Fig.10

Test substance

## INTERNATIONAL SEARCH REPORT

International application No.

**PCT/JP2021/039818**

### A. CLASSIFICATION OF SUBJECT MATTER

*A61L 27/14*(2006.01)i; *A61L 27/18*(2006.01)i; *A61L 27/40*(2006.01)i; *A61L 27/52*(2006.01)i; *A61L 27/56*(2006.01)i
FI: A61L27/14; A61L27/18; A61L27/52; A61L27/56; A61L27/40

According to International Patent Classification (IPC) or to both national classification and IPC

### B. FIELDS SEARCHED

Minimum documentation searched (classification system followed by classification symbols)

A61L27/14; A61L27/18; A61L27/40; A61L27/52; A61L27/56

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

CAplus/MEDLINE/EMBASE/BIOSIS (STN), JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
|---|---|---|
| X | WO 2019/044990 A1 (FUJIFILM CORP) 07 March 2019 (2019-03-07) claim 1, paragraphs [0087]-[0088], [0122], [0127] | 1-7, 9, 12-14, 17-18 |
| Y | | 11, 17, 19-20 |
| A | | 8, 10, 15-16 |
| X | WO 2019/106996 A1 (HITACHI LTD) 06 June 2019 (2019-06-06) claims 1, 3-6, paragraphs [0015]-[0017]. [0045] | 1-8, 10 |
| Y | | 11, 15-17, 19-20 |
| A | | 9, 12-14, 18 |
| Y | KR 10-2015-0136673 A (INHA-INDUSTRY PARTNERSHIP INSTITUTE) 08 December 2015 (2015-12-08) claim 1, paragraph [0005] | 11, 20 |
| Y | WO 2012/133631 A1 (KURARAY CO., LTD.) 04 October 2012 (2012-10-04) claim 1 | 15-16 |

☐ Further documents are listed in the continuation of Box C.    ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **18 November 2021** | **07 December 2021** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

EP 4 238 591 A1

INTERNATIONAL SEARCH REPORT
Information on patent family members

International application No.

**PCT/JP2021/039818**

| Patent document cited in search report | | | Publication date (day/month/year) | Patent family member(s) | | | Publication date (day/month/year) |
|---|---|---|---|---|---|---|---|
| WO | 2019/044990 | A1 | 07 March 2019 | US claims, paragraphs [0145], [0146], [0180], [0206], [0212] | 2020/0246760 | A1 | |
| | | | | EP | 3677287 | A1 | |
| | | | | CN | 111032099 | A | |
| WO | 2019/106996 | A1 | 06 June 2019 | US claims 1, 3-6, paragraphs [0025]-[0027], [0055] | 2020/0360566 | A1 | |
| | | | | EP | 3719112 | A1 | |
| KR | 10-2015-0136673 | A | 08 December 2015 | (Family: none) | | | |
| WO | 2012/133631 | A1 | 04 October 2012 | US claim 1 | 2014/0014573 | A1 | |
| | | | | EP | 2692405 | A1 | |
| | | | | CN | 103458987 | A | |

Form PCT/ISA/210 (patent family annex) (January 2015)

27

**EP 4 238 591 A1**

**Patent documents cited in the description**

- JP 2020180919 A